# EUROPEAN PATENT SPECIFICATION

(11) **EP 4 031 107 B1**
(45) Date of publication and mention of the grant of the patent: **15.07.2026**
(21) Application number: 20780111.9
(22) Date of filing: 17.09.2020
(51) Int. Cl.: A61K 9/00, A61K 47/02, A61K 47/38, A61K 36/00, A61K 9/70, A61K 9/14

(54) **NICOTINE POUCH**
NIKOTINBEUTEL
POCHE À NICOTINE

(30) Priority: 18.09.2019 SE 1951054
(43) Date of publication of application: 27.07.2022
(73) Proprietor: Enorama Pharma AB, 211 34 Malmö (SE)
(72) Inventor: LISSNER, Lucas, 223 59 Lund (SE); FALK, Yana, Z, 212 14 Malmö (SE)
(74) Representative: AWA Sweden AB
(86) International application number: PCT/EP2020/075997
(87) International publication number: WO 2021/053078

(56) References cited:
- WO-A1-2018/233795
- WO-A1-2019/110073
- WO-A1-2020/244721
- US-A1- 2018 193 272
- US-A1- 2019 083 393
- US-B2- 9 114 075
- US-B2- 9 446 004

## Description

### Field of the invention

The present invention relates to pouches for oral delivery of nicotine.

### Technical Background

Smoking of tobacco is associated with health hazards which are not related to the administration of nicotine per se. While nicotine is highly addictive, the most important risk associated with tobacco use come from the multitude of substances formed and released during the combustion of tobacco, e.g. carcinogens such as nitrosamines and various tar components.

It is generally accepted that the difficulty to quit smoking results from smokers being addicted to the constituent nicotine. It is therefore desirable to use alternative means of delivering nicotine to facilitate reduction of or cessation from smoking. Such products should provide nicotine with no or minimal content of other potential hazardous components associated with tobacco use. A number of alternative nicotine administration forms are known such as gum, patch, nasal or oral spray, lozenge and oral pouch. For these products the purity of the constituent nicotine is important for potential toxicity aspects. Also, the taste of the product is clearly important for consumer acceptance and preference.

During the smoking of a cigarette, nicotine is readily absorbed into the smoker's blood via the large surface of the lungs, and it starts affecting the brain within 10 seconds. The result is increased levels of epinephrine as well as dopamine, a neurotransmitter controlling the brain's pleasure center leading to, among other things, "smoker's satisfaction". In contrast, products such as nicotine gum, patch, spray, lozenge and pouch release the nicotine in the oral cavity where it may be absorbed quickly through the oral mucosa or it may be partially swallowed leading to a slower and less efficient absorption via the stomach. For these products, the speed as well as the place of nicotine release (oral mucosa vs stomach) are obviously important. Even for a focused, optimized release of nicotine to the oral mucosa, the onset of action will still be in the order of minutes rather than seconds as facilitated by absorption via the lung.

In addition to taste, purity and the onset of action discussed above, the chemical stability of the nicotine molecule is important for its performance in the oral delivery system. In its free base form nicotine is volatile and rapidly undergoes oxidation leading to discoloration and partial loss of activity. To deal with these issues, nicotine base is sometimes absorbed to appropriate carriers and/or non-covalently bound in salts or in complex with ion exchange resins. While this improves the stability of the constituent nicotine, it obviously also affects the rate of nicotine release from the delivery system. Absorption of the nicotine molecule across the oral mucosa requires the neutral form which predominate only at pH values above 8. Consequently, most delivery systems contain a pH controlling substance such as a buffer which brings the pH into the desired range once the nicotine is released from the delivery system. The rate of local pH adjustment in the mouth becomes yet another parameter deciding the overall performance of the nicotine delivery system.

US patent 9,402,810 describes a pouch based on nicotine extracted from tobacco and subsequently stabilized in the form of a tartrate salt. The pouch described in US patent 9,907,748 is also based on nicotine extracted from tobacco, in this case stabilized via binding to polacrilex resin. However, for stability reasons, US 9,907,748 teaches that the pH controlling agent is being sorbed onto the inactive cellulose carrier to avoid contact with the nicotine salt in the dry pouch. Further, WO 2018/233795 also uses nicotine extracted from tobacco, and a sugar alcohol sweetener is often used as carrier for the nicotine.

One aim of the present invention is to provide an improved nicotine pouch that at least overcomes some of the disadvantages with the products currently on the market.

### Summary of the invention

The stated purpose above is achieved by

a pouch according to the invention, comprising a product for oral delivery of nicotine comprising a powder composition, wherein the powder composition comprises: a nicotine source;
at least one pH adjusting agent; and
at least one filler,
wherein the pouch is permeable to saliva and to components of the powder composition once dissolved in saliva,
wherein the weight proportions of the powder composition are;
0.15-2 wt% of the nicotine source calculated as nicotine base,
2-15 wt% of the pH adjusting agent and
40-85 wt% of the at least one filler, and
wherein said product comprises microcrystalline cellulose in the amount of 30-70 wt%.

In relation to the above it should be emphasized that none of the pouches described in US 9,402,810 or US 9,907,748 are providing an optimal combination of nicotine stability, release and uptake profile. US 9,402,810, uses nicotine in the form of a salt. In contrast, nicotine bound to an ionexchange resin facilitates controlled release and help maximizing the fraction of the dose being absorbed via the mucous membranes in the mouth rather than being swallowed. US 9,907,748 does employ nicotine bound to polacrilex resin. However, in order to minimize contact between nicotine polacrilex (NPR) and buffer in the dry pouch, the buffer is sorbed onto the inactive cellulose carrier. In the presence of saliva, this will prolong the time taken before the buffer makes contact with the nicotine polacrilex. As shown herein, the pouch described in US 9,907,748 releases nicotine relatively slow and more incomplete over a 30-minutes window.

Further, the pouch described in WO 2018/233795A1 uses an excess of polyalcohols which act as the nicotine carrier and also to increase the water solubility of the composition in the pouch. Since it will be difficult to control the water supply in form of saliva for each end-user the high amounts of soluble carrier will possibly lead to loss of nicotine to the stomach due to swallowing rather than absorption through the mucous membrane in the mouth.

In WO 2018/233795A1, a release modifier such as magnesium stearate is needed to slow the release of nicotine from the compositions containing high levels of polyalcohols. In contrast, the proportion of microcrystalline cellulose and (lower level) polyalcohols used herein allows for a controlled nicotine release without the need for specific release modifiers such as magnesium stearate.

In contrast, the proportion of microcrystalline cellulose and much lower level of polyalcohols used herein allows for a controlled nicotine release without the need for specific release modifiers such as magnesium stearate.

In one embodiment the present invention relates to a product wherein the nicotine source has been derived by extraction from tobacco or by chemical synthesis. The nicotine source according to the present invention may be stabilized in the form of a nicotine salt or bound to an ion exchange resin.

In one embodiment the present invention the ion exchange resin may be a weak cation exchange resin, such as polacrilex.

In one embodiment of the present invention the nicotine source may be nicotine polacrilex.

In another embodiment of the present invention the nicotine source may be nicotine benzoate or nicotine maleate.

In a further embodiment the present invention relates to a product wherein the at least one pH adjusting agent is a buffer comprising carbonates, bicarbonates, borates, glycinates, ammonium, phosphates, hydroxides, tris, or mixtures thereof. The pH adjusting agent of the present invention may be a buffer comprising sodium carbonate, sodium bicarbonate, potassium carbonate, or mixtures thereof.

In yet another embodiment the present invention relates to a product wherein the at least one filler comprises polyalcohols, sweeteners, polysaccharides, cellulose, microcrystalline cellulose, natural fibers, flavors, or mixtures thereof. In one embodiment of the present invention the at least one filler may comprise a microcrystalline cellulose, such as Avicel PH-200.

In another embodiment the at least one filler may comprise a mixture of microcrystalline cellulose 90M(102) and the polysaccharide sodium alginate.

The sweeteners of the present invention may be selected from the group consisting of mannitol, xylitol, maltitol, sucralose, acesulfam potassium, aspartame, steviol glycosides, or mixtures thereof. Further, the flavors of the present invention may be selected from the group consisting of menthol, cinnamon, mint, peppermint, spearmint, apple, cherry, melon, mango, peach, passion fruit, orange, blood orange, grape fruit, mandarin, tangerine, licorice, ginger, vanilla, wintergreen, lemon, lime, strawberry, raspberry, salmonberry, lingonberry, cranberry, blueberry, muscadine berry, sea buckthorn, chocolate, coffee, mocha, or mixtures thereof.

In some embodiments, the pouch material may comprise additionally added moisture components in the form of water, propylene glycol, glycerin, or mixtures thereof. In the present invention all reported weight proportions of powder components are based on the total weight (including added moisture components if any).

In one embodiment the present invention relates to a product wherein the weight proportions of the powder composition are; 0.5-2 wt% of the nicotine source calculated as nicotine base, 4-15 wt% of the pH adjusting agent and 70-85 wt% of the at least one filler. The product of the present invention may comprise microcrystalline cellulose in the amount of wt%, preferably wt%. Further, the product of the present invention may comprise nicotine in an amount ranging from 0.5-2 wt% calculated as nicotine base. Furthermore, the product of the present invention may comprise at least one pH adjusting agent in an amount 4-15 wt%, preferably 5-13 wt%, more preferably 5-11 wt%.The product of the present invention may comprise at least one polyalcohol in an amount of 5-50 wt%, preferably 6-45 wt%, more preferably 8-40 wt%. The weight proportions of the nicotine source in relation to the amount of the pH adjusting agent and fillers, especially the microcrystalline cellulose, is of great importance in order to achieve the optimum nicotine release from the pouch.

In another embodiment the present invention relates to a product wherein the weight proportions of the powder composition are; 0.15-2 wt% of the nicotine source calculated as nicotine base, 2-15 wt% of the pH adjusting agent and 40-85 wt% of the at least one filler. The product of the present invention comprises microcrystalline cellulose in the amount of 30-70 wt%. Further, the product of the present invention may comprise nicotine in an amount ranging from 0.15-2 wt% calculated as nicotine base. Furthermore, the product of the present invention may comprise at least one pH adjusting agent in an amount 1-15 wt%, preferably 2-11 wt%.The product of the present invention may optionally comprise at least one polyalcohol in an amount of 0-50 wt%, preferably 0-40 wt%, moisturizing agents such as propylene glycol and/or glycerin in the amount of 0-15wt% and water in the amount of 0-35%.

In one preferred embodiment the present invention relates to a product wherein the at least one filler comprises microcrystalline cellulose, such as Avicel PH-200 or Hicel 90M (102).

In one preferred embodiment the present invention relates to a product wherein the weight proportions of the powder composition are; 0.15-2 wt% of the nicotine source calculated as nicotine base, 2-15 wt% of the pH adjusting agent and 40-85 wt% of the at least one filler.

In one preferred embodiment the present invention relates to a product wherein the product comprises microcrystalline cellulose in the amount of 30-80 wt%.

In one preferred embodiment the present invention relates to a product wherein the pouch has a nicotine release fraction of 50-100 % after 30 min.

Further the invention also relates to a product wherein the pouch is made of a non-woven material. In another embodiment the pouch is made from a biodegradable material. In a preferred embodiment the pouch is made from a home compostable material.

Another aspect of the present invention relates to a method for preparing the pouch according to the present invention, wherein it comprises the steps of:
a) providing the nicotine source, the at least one pH adjusting agent and the at least one filler, in powder form;
b) mixing the powders provided in step a);
c) optionally sieving the powder mixture of step b) into a powder composition; and
d) optionally filling the powder composition into a pouch.

Another aspect of the present invention relates to a method for preparing the pouch according to the present invention, wherein it comprises the steps of:
a) providing the nicotine source, the at least one pH adjusting agent and the at least one filler, in powder form;
b) mixing the powders provided in step a);
c) optionally sieving the powder mixture of step b) into a powder composition; and
d) optionally filling the powder composition into a pouch.
e) optionally addition of the moisture components to step b) in the form of water, propylene glycol, glycerin, or mixtures thereof.

In a further aspect the present invention relates the non-therapeutic use of the pouch according to the present invention for oral delivery of nicotine.

### Short description of the drawings

In the figures below, the nicotine release from products according to the disclosed invention as well as reference products are presented.

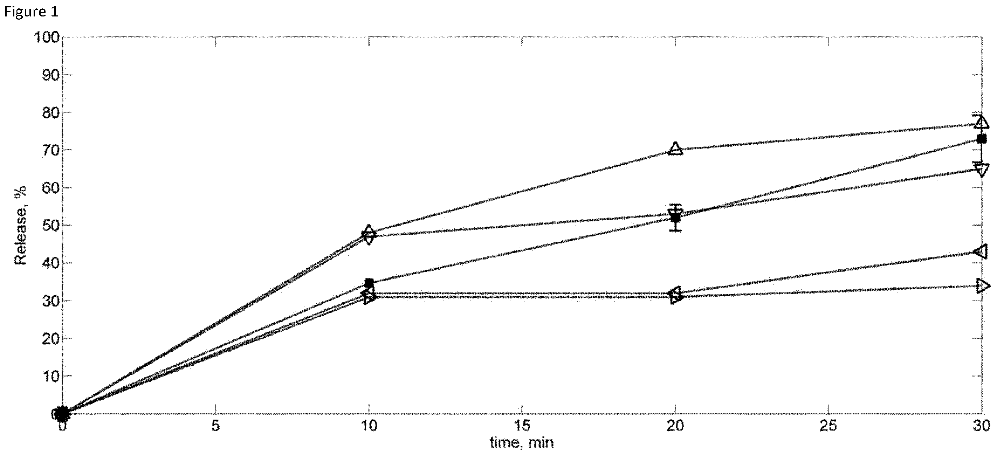

Figure 1 shows nicotine release profiles for the pouch products according to the present invention: Example 1 (Batch A, filled squares); Reference 1A (2 mg Nicotine, left triangles) and Reference 1B (4 mg Nicotine, right triangles); Reference 2A (3 mg Nicotine, up triangles) and Reference 2B (6 mg nicotine, down triangles).

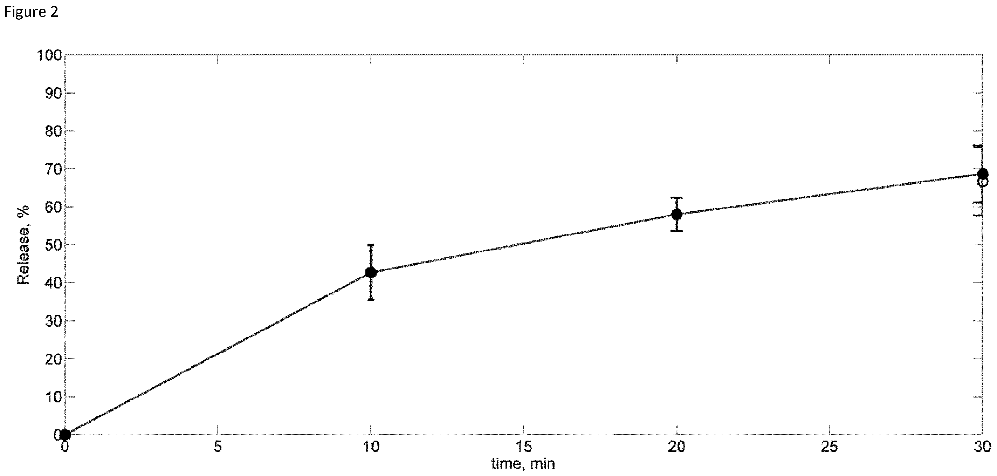

Figure 2 shows nicotine release data for the pouch product according to the present invention, Example 1 (Batch B, filled circles with error bars) and stability data: 1 month, T= 21-22 °C, 60-62 %RH (black empty circle with corresponding error bars).

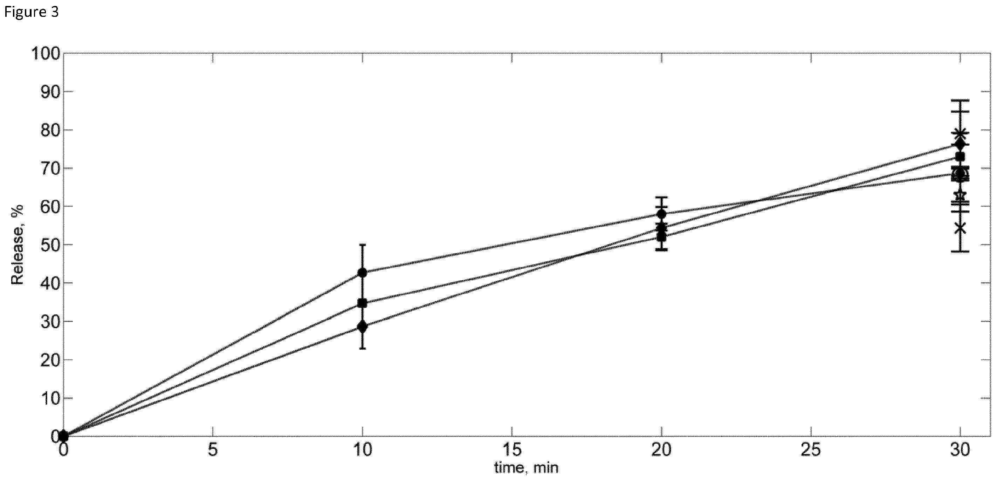

Figure 3 shows nicotine release data for the analyzed pouch products from different batches (A, B and C) according to the present invention, Example 1. Pouch products with different concentration range for the buffer agents (Example 2, black star with error bars), nicotine polacrilex (Example 3, black cross with error bars) and fillers (Example 4, black pentagram with error bars) are analyzed and compared for the nicotine release at 30 min.
Figure 3 shows nicotine release data for the analyzed pouch products with different fillers and moisturizing agents (Example 5, open circle with error bars) are analyzed and compared for the nicotine release at 30 min.

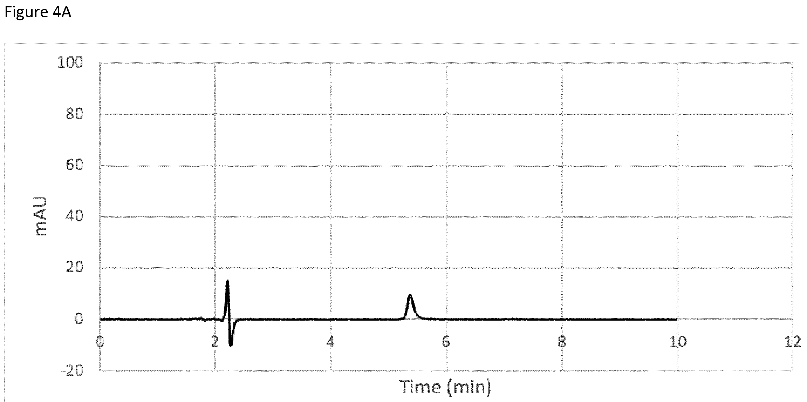

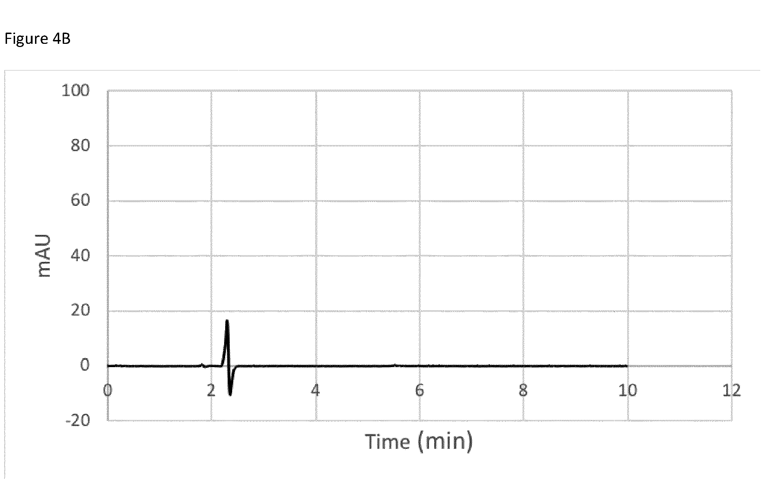

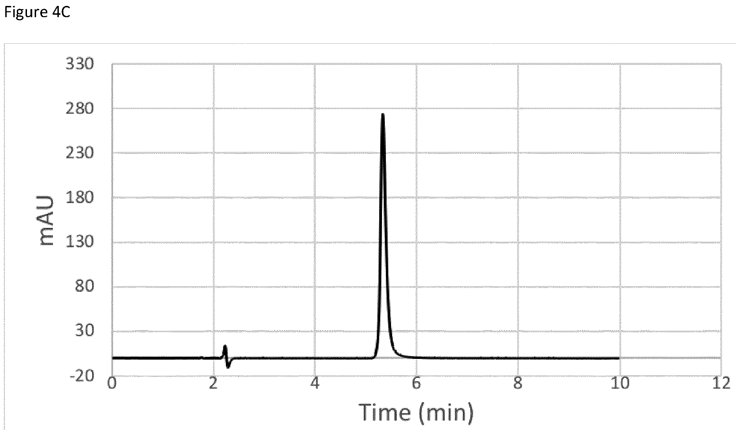

Figures 4A-C show the results from the degradation study of the pouch of the present invention, the chromatograms from the HPLC-UV analysis of the pouch sample stored at 21-22°C / 60-62 %RH for up to 3 months (Figure 4A), sample with the same excipients but without nicotine API (Figure 4B) and sample of nicotine polacrilex 20% API (Figure 4C).

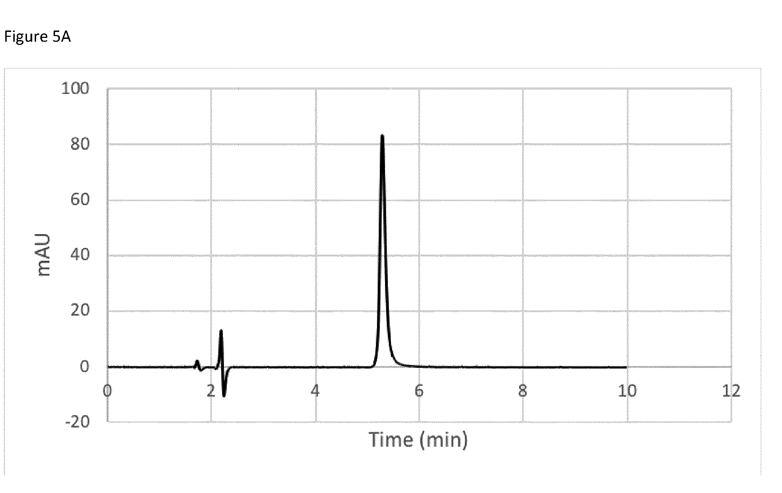

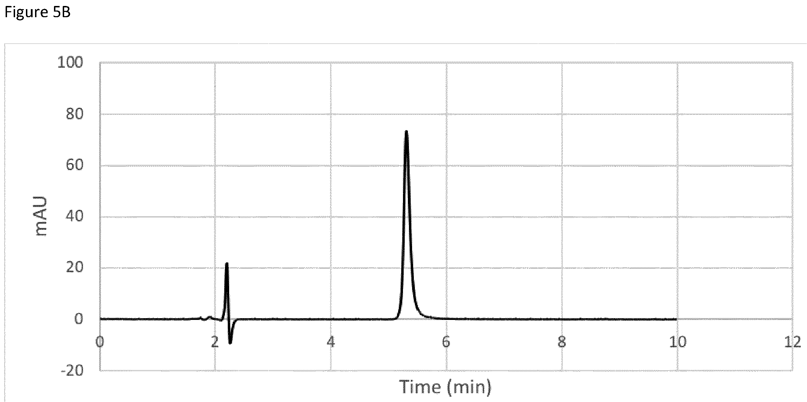

Figures 5A-B show the results from the degradation study of the pouch of the present invention, the chromatograms from the HPLC-UV analysis of a pre-stressed sample of nicotine (Figure 5A) was compared to the chromatogram of nicotine bitartrate dihydrate spiked into the reference sample without API (Figure 5B).

### Detailed description of the invention

Although individual features may be included in different embodiments, these may possibly be combined in other ways, and the inclusion in different embodiments does not imply that a combination of features is not feasible. In addition, singular references do not exclude a plurality. In the context of the present invention, the terms "a", and "an" do not preclude a plurality.

The design of a nicotine delivering pouch require co-optimization of several properties, in particular nicotine stability and release as well as taste masking. Surprisingly, it has been found that a pouch based on nicotine polacrilex according to the present invention is both stable and can release nicotine as fast as a pouch based on a typical salt such as nicotine tartrate. Polacrilex is an insoluble, weakly acidic cation exchange resin. The matrix contains methacrylic acid capable of exchanging hydrogen for nicotine cations, e.g. Amberlite IRP64^{®} or Doshion P-551^{®}.

The pouch according to the present invention contains a powdered mix of a nicotine source, at least one pH controlling agent and at least one filler, such as microcrystalline cellulose, as well as sweeteners and flavors.

In a preferred embodiment according to the present invention, the buffer powder is directly mixed with the other powder components of the pouch to facilitate rapid pH adjustment upon contact with the saliva.

Nicotine may be derived either by extraction from tobacco or from chemical synthesis. In comparison with the synthetic nicotine, the extracted material contains small amounts of tobacco-derived non-nicotine alkaloids . While these impurities may be toxic, they likely contribute difficulties for the taste masking of the product. Although both sources of nicotine are acceptable embodiments of this invention, the synthetic material provides additional advantages in terms of taste setting. Hence, in one preferred embodiment the nicotine is derived from chemical synthesis. In an advantageous embodiment of the present invention the purity of nicotine is in the range of 99.00 - 99.99%, preferably in the range of 99.60 - 99.99%.

According to one embodiment of the present nicotine may be in the form of nicotine polacrilex or a nicotine salt, such benzoate or maleate. In another embodiment nicotine polacrilex is preferred due to the favorable combination of nicotine stability and fast release affected by the direct mix with the pH controlling agent.

Further, the pH controlling agent according to the present invention may be a carbonate buffer, such as sodium carbonate, sodium bicarbonate, potassium carbonate, or mixtures thereof. In other preferred embodiments the buffer may be a borate, such as sodium tetraborate; phosphate, such as mono-, di- or trihydrogenphosphate; or tris(hydroxymethyl)aminomethane (tris). Preferred buffer agents according to the present invention are mixtures of sodium carbonate and sodium bicarbonate.

In addition to the nicotine source and pH controlling agents, the pouch of the present invention also contains at least one filler.

Fillers according to the present invention may include polyalcohols, sweeteners, polysaccharides, cellulose, microcrystalline cellulose, natural fibers, flavors. Polyalcohols, or sugar alcohols are a class of polyols that are commonly obtained by the hydrogenation of sugars. Further, the polyalcohols comprise a lower caloric content than sugars and are also, in general, less sweet. Polysaccharides are polymeric carbohydrate molecules composed of long chains of monosaccharide units bound together by glycosidic linkages, and on hydrolysis give the constituent monosaccharides or oligosaccharides. They range in structure from linear to highly branched. Examples include storage polysaccharides such as starch and glycogen, and structural polysaccharides such as cellulose, chitin and alginate. Cellulose is a very common natural polymer and it is the largest component of plant cell walls. The cellulose usually consists of polymer chains that are 10,000-15,000 molecules long. Microcrystalline cellulose (MCC) is synthetically made and consists of chains that are between 100 and 300 molecules long. With natural fibers means fibers that are produced by plants, animals, or geological processes.

The at least one filler of the present invention may be microcrystalline cellulose, such as Avicel PH-200, which may have solubility of less than 5 g/100 ml. Fillers with poor water solubility limit the water solubility of the powder composition in the pouch and, therefore, do not allow the pouch content to be completely dissolved when in contact with water supplied in the form of saliva. Thus, the use of fillers with poor water solubility, such as Avicel PH-200, may result in a relativley slow release and direct absorbtion of nicotine through the mucus membrane in the mouth. In a preferred embodiment the water solublility of microcrystalline cellulose is less than 2.5 g /100 ml, more preferable less than 1.0 g/100 ml, even more preferable less than 0.75 g/100 ml, most preferable less than 0.58 g/100 ml measured in H₂O at 25 °C and pH 7. This contributes greatly to the advantagous nicotine release profile of the pouch according to the present invention.

The at least one filler in one embodiment of the present invention may be microcrystalline cellulose, such as Avicel PH-200. In another embodiment of the present invention, the at least one filler may comprise a mixture of microcrystalline cellulose 90M(102) and the polysaccharide sodium alginate. Both embodiments of the present invention may have a water solubility of less than 5 g/100 ml. Fillers with poor water solubility limit the water solubility of the powder composition in the pouch and, therefore, do not allow the pouch content to be completely dissolved when in contact with water supplied in the form of saliva. Thus, the use of fillers with poor water solubility, such as Avicel PH-200 or Hicel 90M(102), may result in a relativley slow release and direct absorbtion of nicotine through the mucus membrane in the mouth. In a preferred embodiment the water solublility of microcrystalline cellulose is less than 2.5 g /100 ml, more preferable less than 1.0 g/100 ml measured in H₂O at 25 °C. This contributes greatly to the advantagous nicotine release profile of the pouch according to the present invention.

Sweeteners according to the present invention may comprise mannitol, xylitol, maltitol, sucralose, acesulfam potassium, aspartame, steviol glycosides, or mixtures thereof. Flavors according to the present invention may include menthol, cinnamon, mint, peppermint, spearmint, apple, cherry, melon, mango, peach, passion fruit, orange, blood orange, grape fruit, mandarin, tangerine, licorice, ginger, vanilla, wintergreen, lemon, lime, strawberry, raspberry, salmonberry, lingonberry, cranberry, blueberry, muscadine berry, sea buckthorn, chocolate, coffee, mocha, or mixtures thereof.

In an advantagous embodiment of the present invention, the distinct balance of microcrystalline cellulose and polyalcohol levels contribute to the favorable release profile of nicotine from the matrix.

### Examples

The formulation of the present invention is exemplary and should be considered as an inspiration to obtain a similar outcome within the scope of the present invention. A specification of suppliers for the different ingredients used in the present application is disclosed in Table 1.

A specification of suppliers for the different ingredients used in the present application is disclosed in Table 1B.

**Table 1. Specification of suppliers for the different ingredients.**

| Ingredients | Supplier | Form | Particle size | Solubility in water | Function |
|---|---|---|---|---|---|
| Nicotine Polacrilex (NPR) 20% | Siegfried | Solid/ white fine powder | 96%: < 710 µm | Insoluble | Nicotine source |
| | | | 90%: < 210 µm | | |
| Xylitol | Sigma-Aldrich | Solid/ white crystalline powder | - | Very soluble 642 mg/ml at 25°C | Filler Sweetener |
| D-Mannitol | Sigma-Aldrich | Solid/ white crystalline powder | - | Very soluble 216 mg/ml at 25°C | Filler Sweetener |
| Avicel PH-200 Microcrystalline cellulose | FMC | Solid/ white free-flowing powder | 200 µm | Insoluble (water soluble substances: NMT 12.5 mg/5g (0.25%) at 25°C) | Filler |
| Sodium Carbonate Monohydrate | Sigma-Aldrich | Solid/ white powder | - | Very soluble 307 mg/ml at 25°C | Buffering agent |
| Sodium Bicarbonate | Sigma-Aldrich | Solid/ white powder | | 103 mg/ml at 25°C | Buffering agent |
| L-Menthol | Sigma-Aldrich | Solid/ transparent large crystals | - | Low soluble 0.49mg/ml at 25°C | Flavoring agent |
| Apple flavor | Firmenich | Solid/ colored powder | - | - | Flavoring agent |
| Cinnamon flavor | Firmenich | Solid/ colored powder | - | - | Flavoring agent |
| Acesulfame K | Celanese | Solid/ white crystalline powder | Min 95%: <1000 µm | High solubility: 270 mg/ml at 20°C | Sweetener |

**Table 1B. Specification of suppliers for the different ingredients.**

| Ingredients | Supplier | Form | Particle size | Solubility in water | Function |
|---|---|---|---|---|---|
| Nicotine Polacrilex (NPR) 20% | Siegfried | Solid/ white fine powder | 96%: < 710 µm | Insoluble | Nicotine source |
| | | | 90%: < 210 µm | | |
| Xylitol | Sigma-Aldrich | Solid/ white crystalline powder | - | Very soluble 642 mg/ml at 25°C | Filler Sweetener |
| D-Mannitol | Sigma-Aldrich | Solid/ white crystalline powder | - | Very soluble 216 mg/ml at 25°C | Filler Sweetener |
| Avicel PH-200 Microcrystalline cellulose | FMC | Solid/ white free-flowing powder | 200 µm | Insoluble (water soluble substances: NMT 12.5 mg/5g (0.25%) at 25°C) | Filler |
| Hicel 90M (102) Microcrystalline cellulose | Brenntag | Solid/ white free flowing powder | 90 µm | Insoluble (water soluble substances: <0.24%) | Filler |
| Satialgine S 900 NS Sodium Alginate | Brenntag | Solid/ creamy-white to light-brown powder | - | Soluble | Filler |
| Sodium Carbonate Monohydrate | Brenntag | Solid/ white powder | - | Very soluble 307 mg/ml at 25°C | Buffering agent |
| Sodium Bicarbonate | Sigma-Aldrich | Solid/ white powder | | 103 mg/ml at 25°C | Buffering agent |
| L-Menthol | Sigma-Aldrich | Solid/ transparent large crystals | - | Low soluble 0.49mg/ml at 25°C | Flavoring agent |
| Menthol flavor | Firmenich | Solid powder | - | - | Flavoring agent |
| Flavors | Firmenich | Solid/ white or colored powder | - | - | Flavoring agent |
| Acesulfame K | Celanese | Solid/ white crystalline powder | Min 95%: <1000 µm | High solubility: 270 mg/ml at 20°C | Sweetener |
| Ammonium Chloride | Brenntag | White crystalline powder | - | - | Taste enhancer |
| Propylene glycol | Caldic | Clear colorless odorless liquid | - | Soluble | Moisturizing agent |

### Product manufacturing

A product with a composition according to the present invention is manufactured in the following manner: all ingredients, that are in powder form, are mixed and sieved into the final powder mixture. The resulting powder is filled into a pouch made of a non-woven material or cloth. Non-woven material, cloths or fabrics may be defined as sheet or web structures bonded together by entangling fibers or filaments, and by perforating films, mechanically, thermally, or chemically. They are flat, porous sheets that are made directly from separate fibers or from molten plastic or plastic film. They are not made by weaving or knitting and do not require converting the fibers to yarn. The whole manufacture process is performed at ambient conditions of temperature and relative humidity (RH), ranging between 21-25 °C and 30-60 %RH, respectively.

### Solubility in water

The water used for solubility measurements is either destilled or filtered via a Millipore Milli-Q system. An unused pouch with weight P0 (g) is placed in a test tube containing 20 ml of water and left on the shaker at room temberature for 1 h. The pouch is isolated and dried to constant weight Pe (g) in air at room tempetature. Three replicates for each batch are used in the solubility measurements. The water solubility of the pouch composition is then (P0-Pe) (g) per 20 ml water.

### In-Vitro release experiments

An experimental method for the determination of the nicotine release from pouch was performed using UV spectrophotometric methodology. Pouch sample preparation and method of analysis were adapted and modified from the published methods for pouch analysis (see US 9,402,810).

Briefly, a pouch sample is placed on the middle of the filter paper which is pre-soaked in 20 ml water phase in a Petri dish. The whole setup is covered by lid to maintain the relative humidity level and prevent water evaporation. As the the pouch starts to absorb the water, the nicotine polacrilex is dissolved by liquid and the released nicotine diffuses into the external water phase.

This is conducted at specific incubation times, i.e. 10, 20 and 30 min (note a new pouch for each time). Three replicates from each batch of Example 1 are used in the experiment for each time. After each analyzed timepoint, the pouch sample is placed into the test tube containing 15 ml of water and left on the shaker for 1.5-2 hours.

A number of unused pouches from the same batch are also placed into the test tube, containing 15 ml purified water, and left on the shaker for 1.5-2 hours for analysis of the total nicotine content.

Afterwards, the water phase contaning nicotine is analyzed for the residual content of nicotine using the UV spectrophotometer (Shimadzu, UV1800). First, each sampling of the water phase from the test tube is filtered using the 0.45 µm filter unit. The filtered solution is then diluted to the suitable concentration, and the sample absorbance is measured. The measurements are conducted at photometric mode with fixed wavelength of 260 nm.

The nicotine concentration for the analyzed samples (non-used and used pouches) is calculated from a calibration curve, and the total and residual nicotine content is determined. The release of nicotine at specific time is calculated by substracting the 'residual nicotine content' from the total nicotine content' and dividing it by the 'total nicotine content'. Table 2 shows four different example compositions (Examples 1-4) according to the present invention that have been analyzed. Table 3 summarizes the obtained data for the nicotine release for the four example compositions of the present invention and reference pouch samples. The obtained data are plotted and shown in Figures 1-3.

Table 2B shows five different example compositions (Examples 1-5) according to the present invention that have been analyzed. Table 3B summarizes the obtained data for the nicotine release for the five example compositions of the present invention and reference pouch samples. The obtained data are plotted and shown in Figures 1-3.

**Table 2. Formulation compositions of the present invention. The pouch weight is 0.4 g. The nicotine dose corresponds to the 4 mg per pouch (Examples 1, 2 and 4) and 8 mg per pouch (Example 3).**

| Content | Example 1 | Example 2 | Example 3 | Example 4 |
|---|---|---|---|---|
| NPR 20%* | 5 | 5 | 10 | 5 |
| Xylitol | 16 | 16 | 16 | 4 |
| Mannitol | 16 | 16 | 16 | 4 |
| Avicel PH-200 | 48 | 53.2 | 43 | 72 |
| Sodium Carbonate | 4.0 | 2.0 | 4.0 | 4.0 |
| Sodium Bicarbonate | 6.4 | 3.2 | 6.4 | 6.4 |
| Menthol | 0.2 | 0.2 | 0.2 | 0.2 |
| Apple flavor | 2.0 | 2.0 | 2.0 | 2.0 |
| Cinnamon flavor | 2.14 | 2.14 | 2.14 | 2.14 |
| Ace K** | 0.26 | 0.26 | 0.26 | 0.26 |

| | | | | |
|---|---|---|---|---|
| *NPR - Nicotine polacrilex; **Ace K - Acesulfame potassium. | | | | |

**Table 2B. Formulation composition of the present invention. The pouch weight is 0.4 g (Examples 1 - 4) and 0.6 g (Example 5). The nicotine dose corresponds to the 4 mg per pouch (Examples 1, 2, 4 and 5) and 8 mg per pouch (Example 3).**

| Content | Example 1 | Example 2 | Example 3 | Example 4 | Example 5 |
|---|---|---|---|---|---|
| NPR 20%* | 5 | 5 | 10 | 5 | 3.3 |
| Xylitol | 16 | 16 | 16 | 4 | 0 |
| Mannitol | 16 | 16 | 16 | 4 | 0 |
| Sodium Alginate | 0 | 0 | 0 | 0 | 5 |
| Avicel PH-200 | 48 | 53.2 | 43 | 72 | 0 |
| Hicel 90M (102) | 0 | 0 | 0 | 0 | 48.9 |
| Sodium Carbonate | 4.0 | 2.0 | 4.0 | 4.0 | 2.5 |
| Sodium Bicarbonate | 6.4 | 3.2 | 6.4 | 6.4 | 0 |
| Water | 0 | 0 | 0 | 0 | 20.0 |
| Propylene glycol | 0 | 0 | 0 | 0 | 14.5 |
| L-Menthol | 0.2 | 0.2 | 0.2 | 0.2 | 0 |
| Menthol flavor | 0 | 0 | 0 | 0 | 0.7 |
| Apple flavor | 2.0 | 2.0 | 2.0 | 2.0 | 0 |
| Cinnamon flavor | 2.14 | 2.14 | 2.14 | 2.14 | 0 |
| Peppermint flavor | 0 | 0 | 0 | 0 | 3.0 |
| Spearmint flavor | 0 | 0 | 0 | 0 | 1.0 |
| Ace K** | 0.26 | 0.26 | 0.26 | 0.26 | 0.6 |
| Ammoinium chloride | 0 | 0 | 0 | 0 | 0.5 |

| | | | | | |
|---|---|---|---|---|---|
| *NPR - Nicotine polacrilex; **Ace K - Acesulfame potassium. | | | | | |

**Table 3. Pouches according to the present invention together with reference pouches and their respective measured nicotine release at specific timepoints.**

| Sample ID | Nicotine source/ Conc % | Nicotine dose/ pouch mg | Release fraction (%) *X*_{*released*(*time*)} | | |
|---|---|---|---|---|---|
| | | | 10 min | 20 min | 30 min |
| *In- Vitro* | | | | | |
| Example 1 | NPR/ 1.0 % | 4 | 35 | 54 | 75 |
| | | | 34 | 54 | 66 |
| Batch A | | | 35 | 48 | 78 |
| Mean value ± Std | | | 35 ± 0.6 | 52 ± 3.5 | 73 ± 6.2 |
| Example 1 | NPR/ 1.0 % | 4 | 38 | 63 | 61 |
| | | | 51 | 55 | 76 |
| Batch B | | | 39 | 56 | 69 |
| Mean value ± Std | | | 43 ± 7.2 | 58 ± 4.3 | 69 ± 7.5 |
| Stability study: 1 month (T=21-22°C, 60-62 %RH), Batch B | | | - | - | 61 |
| | | | | | 62 |
| | | | | | 77 |
| Mean value ± Std | | | - | - | 67 ± 8.9 |
| Example 1 | NPR/ 1.0 % | 4 | 22 | 60 | 71 |
| | | | 32 | 49 | 72 |
| Batch C | | | 32 | 54 | 86 |
| Mean value ± Std | | | 29 ± 5.8 | 54 ± 5.5 | 76 ± 8.4 |
| Example 2 | NPR/ 1.0 % | 4 | - | - | 69 |
| | | | | | 84 |
| | | | | | 84 |
| Mean value ± Std | | | - | - | 79 ± 8.7 |
| Example 3 | NPR/ 2.0 % | 8 | - | - | 53 |
| | | | | | 49 |
| | | | | | 61 |
| Mean value ± Std | | | - | - | 54 ± 6.1 |
| Example 4 | NPR/ 1.0 % | 4 | - | - | 68 |
| | | | | | 61 |
| | | | | | 60 |
| Mean value ± Std | | | - | - | 63 ± 4.3 |
| Reference 1A (Zonnic) | NPR/ 1.1 % | 2 | 32 | 32 | 43 |
| Reference 1B (Zonnic) | NPR/ 2.2 % | 4 | 31 | 31 | 34 |
| Reference 2A (ZYN mini dry) | Nicotine Salt/ 0.75 % | 3 | 48 | 70 | 77 |
| Reference 2B (ZYN mini dry) | Nicotine Salt/ 1.5 % | 6 | 47 | 53 | 65 |

**Table 3B. Pouches according to the present invention together with reference pouches and their respective measured nicotine release at specific timepoints.**

| Sample ID | Nicotine source/ Conc % | Nicotine dose/ pouch mg | Release fraction (%) | | |
|---|---|---|---|---|---|
| | | | *X*_{*released*(*time*)} | | |
| | | | 10 min | 20 min | 30 min |
| *In- Vitro* | | | | | |
| Example 1 | NPR/ 1.0 % | 4 | 35 | 54 | 75 |
| Batch A | | | 34 | 54 | 66 |
| | | | 35 | 48 | 78 |
| Mean value ± Std | | | 35 ± 0.6 | 52 ± 3.5 | 73 ± 6.2 |
| Example 1 | NPR/ 1.0 % | 4 | 38 | 63 | 61 |
| Batch B | | | 51 | 55 | 76 |
| | | | 39 | 56 | 69 |
| Mean value ± Std | | | 43 ± 7.2 | 58 ± 4.3 | 69 ± 7.5 |
| Stability study: 1 month (T=21-22°C, 60-62 %RH), Batch B | | | - | - | 61 |
| | | | | | 62 |
| Mean value ± Std | | | - | - | 67 ± 8.9 |
| Example 1 | NPR/ 1.0 % | 4 | 22 | 60 | 71 |
| Batch C | | | 32 | 49 | 72 |
| | | | 32 | 54 | 86 |
| Mean value ± Std | | | 29 ± 5.8 | 54 ± 5.5 | 76 ± 8.4 |
| Example 2 | NPR/ 1.0 % | 4 | - | - | 69 |
| | | | | | 84 |
| | | | | | 84 |
| Mean value ± Std | | | - | - | 79 ± 8.7 |
| Example 3 | NPR/ 2.0 % | 8 | - | - | 53 |
| | | | | | 49 |
| | | | | | 61 |
| Mean value ± Std | | | - | - | 54 ± 6.1 |
| Example 4 | NPR/ 1.0 % | 4 | - | - | 68 |
| | | | | | 61 |
| | | | | | 60 |
| Mean value ± Std | | | - | - | 63 ± 4.3 |
| Example 5 | NPR/ 0.66 % | 4 | - | - | 67 |
| | | | | | 68 |
| | | | | | 70 |
| Mean value ± Std | | | - | - | 68 ± 1.5 |
| Reference 1A (Zonnic) | NPR/ 1.1 % | 2 | 32 | 32 | 43 |
| Reference 1B (Zonnic) | NPR/ 2.2 % | 4 | 31 | 31 | 34 |
| Reference 2A (ZYN mini dry) | Nicotine Salt/ 0.75 % | 3 | 48 | 70 | 77 |
| Reference 2B (ZYN mini dry) | Nicotine Salt/ 1.5 % | 6 | 47 | 53 | 65 |

### 1) Pouch analysis: a comparison with commercially available reference pouches.

Figure 1 shows that the pouch products of the present invention - Example 1 (filled squares with error bars) results in similar nicotine release at 10 min and higher release of nicotine at 20 min and 30 min as compared to the nicotine release for Reference 1 pouch products containing nicotine polacrilex (right and left triangles). Moreover, all the examples of the pouch products according to the present invention show similar release of nicotine as compared to Reference 2 pouch product, containing nicotine salt (up and down triangles) at 20 min and 30 min.

Pouch based on nicotine polacrilex according to the present invention can release nicotine as fast as a pouch based on a typical salt such as nicotine tartrate. Moreover, pouch based on nicotine polacrilex according to the present invention can release higher amount of nicotine in comparison to other nicotine polacrilex based pouch products.

### 2) Stability study:

- nicotine release at 30 min
- degradation product
Pouch samples from Example 1 were stored in a plastic container with closed (but not hermetically sealed) lid at 21-22°C / 60-62 %RH for up to 3 months. The release of nicotine at 30 min for the pouch product stored for 1 month at 21-22°C / 60-62 %RH was analyzed, using the UV spectrophotometrical method described above.

Figure 2 shows the obtained results where no changes in the nicotine release are observed for the pouch product samples (Example 1) stored for one month at 21-22°C / 60-62 %RH (black empty circle).

Pouch samples from Example 1 were also analyzed for the detection of potential degradation products, using HPLC-UV method (Agilent 1200 series HPLC). Samples with the same excipients but without the nicotine API and samples with the nicotine API was used to identify reference (blank) peaks. A pre-stressed sample of nicotine was prepared (using elevated temperature and light) to reveal any peaks originating from heat- and light- induced degradation of nicotine. Figures 4A-C show the chromatograms from the HPLC-UV analysis of the pouch sample of the present invention stored at 21-22°C / 60-62 %RH for up to 3 months (Figure 4A), a pouch sample with the same excipients but without nicotine API (Figure 4B) and a sample of nicotine polacrilex 20% API (Figure 4C). The obtained chromatograms show no peaks for the potential degradation products in analyzed samples. The obtained peaks in the chromatogram correponds to nicotine or solvent injection peak. The chromatogram from the HPLC-UV analysis of pre-stressed sample of nicotine (Figure 5A), which was compared to the chromatogram of nicotine bitartrate dihydrate spiked into the reference sample without API (Figure 5B). No additional peaks from potential degradation products were observed in the chromatogram of the pre-stressed sample (Figure 5A).

Pouch based on nicotine polacrilex according to the present invention is stable and release the same amount of nicotine after 1 month storage as a freshly manufactured pouch based on nicotine polacrilex according to the present invention. Pouch based on nicotine polacrilex according to the present invention is stable and show no potential degradation products in analyzed pouch samples from example 1, stored at 21-22°C / 60-62 %RH for up to 3 months.

### 3) Concentration range for the components:

- Buffer (reduced twice)
- Nicotine polacrilex (increased twice)
- Filler (polyalcohols reduced 4 times)
- Filler (no polyalcohols, addition of polysaccharide and/or moisturizing agents)
Figure 3 shows similar nicotine release at 30 min even if the content of buffer agents is reduced twice in the current pouch formulation recipe (black star with error bars, Example 2).

If the nicotine content is increased twice in the current pouch formulation recipe (Table 2, Example 3), the release of nicotine at 30 min is lower as compared to the release of nicotine of Examples 1, 2 and 4 at 30 min (Table 2). However, similar trend could be observed in the analyzed reference pouch products with higher amount of nicotine (Figure 1, black triangles). For example, analysis of Reference 1B pouch containing 4 mg NPR (Figure 1, right triangles) results in lower nicotine release at 30 min as compared to the Reference 1A pouch containing 2 mg NPR (Figure 1, left triangles). Similar scenario is observed for Reference 2 pouch products: pouch, containing 6 mg nicotine salt (Figure 1, down triangles) shows lower release of nicotine at 30 min than pouch containing 3 mg nicotine salt (Figure 1, up triangles).

Figure 3 shows slightly lower release of nicotine at 30 min if the content of polyalcohols is reduced four times in the current formulation recipe (black pentagram with error bars, Example 4).

Figure 3 shows similar nicotine release even if the content of polyalcohols reduced to zero but with the addition of polysaccharides such as sodium alginate and moisturizing agents (open circle with error bars, Example 5).

Figure 3 also shows batch-to-batch variation according to the present invention, Example 1. There is a relatively high batch-to-batch variation (filled circles, squares and diamonds with error bars) of the nicotine at 10 min.

A pouch based on nicotine polacrilex with reduced amount of buffer agents according to the present invention, Example 3, has the same release of nicotine at 30 min as compared with a pouch based on nicotine polacrilex according to the present invention, Example 1. It appears that a pouch based on nicotine polacrilex with reduced amount of buffer agents according to the present invention, Example 3, has similar release of nicotine at 30 min as compared with a reference pouch based on a nicotine salt such as nicotine tartrate. A pouch based on nicotine polacrilex with reduced amount of polyalcohols according to the present invention, Example 4, has slightly lower release of nicotine at 30 min as compared with a pouch based on nicotine polacrilex according to the present invention, Example 1. A pouch based on nicotine polacrilex with reduced amount of polyalcohols according to the present invention, Example 4, has higher nicotine release as compared with a reference pouch based on nicotine polacrilex.

The pH of the nicotine containing water phase from the test tube for the examined pouch samples of Example 1 was also measured. The obtained pH values are summarized in Table 4.

Table 4 shows that the pH for the pouch based on nicotine polacrilex according to the present invention is above pH 8.

The pH of the nicotine containing water phase from the test tube for the examined pouch samples of Example 1 and Example 5 was also measured. The obtained pH values are summarized in Table 4B.

Table 4B shows that the pH for the pouch based on nicotine polacrilex according to the present invention is above pH 8.

**Table 4. pH values of the water phase containing nicotine in the test tube for the examined pouch samples at specific timepoint.**

| Specific timepoint, Min | pH values |
|---|---|
| *In- Vitro* | |
| 0 (non-used) | 8.62 |
| 10 | 8.47 |
| 20 | 8.42 |
| 30 | 8.49 |

**Table 4B. pH values of the water phase containing nicotine in the test tube for the examined pouch samples at specific timepoint.**

| Specific timepoint, Min | pH values | |
|---|---|---|
| *In- Vitro* | | |
| Examples | 1 | 5 |
| 0 (non-used) | 8.62 | 9.67 |
| 10 | 8.47 | - |
| 20 | 8.42 | - |
| 30 | 8.49 | 9.58 |

## Claims

1. A pouch, comprising
a product for oral delivery of nicotine comprising a powder composition, wherein the powder composition comprises:
a nicotine source;
at least one pH adjusting agent; and
at least one filler,
wherein the pouch is permeable to saliva and to components of the powder composition once dissolved in saliva,
wherein the weight proportions of the powder composition are; 0.15-2 wt% of the nicotine source calculated as nicotine base, 2-15 wt% of the pH adjusting agent and 40-85 wt% of the at least one filler, and
wherein said product comprises microcrystalline cellulose in the amount of 30-70 wt%.

2. The product according to claim 1, wherein the nicotine source has been derived by extraction from tobacco or by chemical synthesis.

3. The product according to claim 1 or 2, wherein the nicotine source is stabilized in the form of a nicotine salt or bound to an ion exchange resin.

4. The product according to claim 3, wherein the ion exchange resin is a weak cation exchange resin, such as polacrilex.

5. The product according to claims 1-3, wherein the nicotine source is nicotine benzoate or nicotine maleate.

6. The product according to any one of the preceding claims, wherein the at least one pH adjusting agent is a buffer comprising carbonates, bicarbonates, borates, glycinates, ammonium, phosphates, hydroxides, tris, or mixtures thereof.

7. The product according to any one of the preceding claims, wherein said at least one filler comprises polyalcohols, sweeteners, polysaccharides, cellulose, natural fibers, flavors, or mixtures thereof.

8. The product according to any one of the preceding claims, wherein the at least one filler comprises microcrystalline cellulose, such as Avicel PH-200.

9. The product according to claim 7, wherein the sweeteners are selected from the group consisting of mannitol, xylitol, maltitol, sucralose, acesulfam potassium, aspartame, steviol glycosides, or mixtures thereof.

10. The product according to claim 7, wherein the flavors are selected from the group consisting of menthol, cinnamon, mint, peppermint, spearmint, apple, cherry, melon, mango, peach, passion fruit, orange, blood orange, grape fruit, mandarin, tangerine, licorice, ginger, vanilla, wintergreen, lemon, lime, strawberry, raspberry, salmonberry, lingonberry, cranberry, blueberry, muscadine berry, sea buckthorn, chocolate, coffee, mocha, or mixtures thereof.

11. The product according to any one of the preceding claims, wherein the weight proportions of the powder composition are; 0.5-2 wt% of the nicotine source calculated as nicotine base, 4-15 wt% of the pH adjusting agent and 70-85 wt% of the at least one filler.

12. The product according to any one of the preceding claims, wherein the pouch has a nicotine release fraction of 50-100 % after 30 min.

13. The product according to any one of the preceding claims, wherein the pouch is made of a non-woven material.

14. A method for preparing the product according to any of claims 1-13, wherein it comprises the steps of:
a) providing the nicotine source, the at least one pH adjusting agent and the at least one filler, in powder form;
b) mixing the powders provided in step a);
c) optionally sieving the powder mixture of step b) into a powder composition; and
d) optionally filling the powder composition into a pouch.

15. Non-therapeutic use of the product according to any of claims 1-13 for oral delivery of nicotine.

## Patentansprüche

1. Beutel umfassend
ein Produkt zur oralen Abgabe von Nikotin, umfassend eine Pulverzusammensetzung, wobei die Pulverzusammensetzung umfasst:
eine Nikotinquelle;
wenigstens ein pH-Einstellmittel; und
wenigstens einen Füllstoff,
wobei der Beutel für Speichelflüssigkeit und für Komponenten der Pulverzusammensetzung, sobald in Speichelflüssigkeit gelöst, durchlässig ist,
wobei die Gewichtsanteile der Pulverzusammensetzung sind: 0,15-2 Gew.-% an der Nikotinquelle, berechnet als Nikotinbase, 2-15 Gew.-% an dem pH-Einstellmittel und 40-85 Gew.-% an dem wenigstens einen Füllstoff, und
wobei das Produkt mikrokristalline Cellulose in der Menge von 30-70 Gew.-% umfasst.

2. Produkt nach Anspruch 1, wobei die Nikotinquelle durch Extraktion aus Tabak oder durch chemische Synthese gewonnen worden ist.

3. Produkt nach Anspruch 1 oder 2, wobei die Nikotinquelle in der Form eines Nikotinsalzes stabilisiert oder an ein Ionenaustauschharz gebunden ist.

4. Produkt nach Anspruch 3, wobei das Ionenaustauschharz ein schwaches Kationenaustauschharz, wie z.B. Polacrilex, ist.

5. Produkt nach Ansprüchen 1-3, wobei die Nikotinquelle Nikotinbenzoat oder Nikotinmaleat ist.

6. Produkt nach einem der vorstehenden Ansprüche, wobei das wenigstens eine pH-Einstellmittel ein Puffer umfassend Carbonate, Bicarbonate, Borate, Glycinate, Ammonium, Phosphate, Hydroxide, Tris oder Gemische davon ist.

7. Produkt nach einem der vorstehenden Ansprüche, wobei der wenigstens eine Füllstoff Polyalkohole, Süßungsmittel, Polysaccharide, Cellulose, natürliche Fasern, Aromen oder Gemische davon umfasst.

8. Produkt nach einem der vorstehenden Ansprüche, wobei der wenigstens eine Füllstoff mikrokristalline Cellulose, wie z.B. Avicel PH-200, umfasst.

9. Produkt nach Anspruch 7, wobei die Süßungsmittel ausgewählt sind aus der Gruppe bestehend aus Mannit, Xylit, Maltit, Sucralose, Acesulfam-Kalium, Aspartam, Steviolglycosiden und Gemischen davon.

10. Produkt nach Anspruch 7, wobei die Aromen ausgewählt sind aus der Gruppe bestehend aus Menthol, Zimt, Minze, Pfefferminze, grüner Minze, Apfel, Kirsche, Melone, Mango, Pfirsich, Passionsfrucht, Orange, Blutorange, Grapefruit, Mandarine, Tangerine, Lakritz, Ingwer, Vanille, Wintergrün, Zitrone, Limette, Erdbeere, Himbeere, Lachsbeere, Preiselbeere, Cranberry, Heidelbeere, Muscadine-Beere, Sanddorn, Schokolade, Kaffee, Mokka und Gemischen davon.

11. Produkt nach einem der vorstehenden Ansprüche, wobei die Gewichtsanteile der Pulverzusammensetzung sind: 0,5-2 Gew.-% an der Nikotinquelle, berechnet als Nikotinbase, 4-15 Gew.-% an dem pH-Einstellmittel und 70-85 Gew.-% an dem wenigstens einen Füllstoff.

12. Produkt nach einem der vorstehenden Ansprüche, wobei der Beutel einen Nikotinfreisetzungsanteil von 50-100 % nach 30 min aufweist.

13. Produkt nach einem der vorstehenden Ansprüche, wobei der Beutel aus einem Vliesmaterial besteht.

14. Verfahren zur Herstellung des Produkts nach einem der Ansprüche 1-13, umfassend die Schritte:
a) Bereitstellen der Nikotinquelle, des wenigstens einen pH-Einstellmittels und des wenigstens einen Füllstoffs in Pulverform;
b) Mischen der bei Schritt a) bereitgestellten Pulver;
c) gegebenenfalls Sieben des Pulvergemischs von Schritt b) zu einer Pulverzusammensetzung; und
d) gegebenenfalls Füllen der Pulverzusammensetzung in einen Beutel.

15. Nichttherapeutische Verwendung des Produkts nach einem der Ansprüche 1-13 zur oralen Abgabe von Nikotin.

## Revendications

1. Poche comprenant
un produit pour administration orale de nicotine comprenant une composition en poudre, la composition en poudre comprenant :
une source de nicotine ;
au moins un agent d'ajustement du pH ; et
au moins une charge,
dans laquelle la poche est perméable à la salive et à des composants de la composition en poudre une fois dissoute dans la salive,
dans laquelle les proportions en poids de la composition en poudre sont ; 0,15-2 % en poids de la source de nicotine calculés en tant que nicotine base, 2-15 % en poids de l'agent d'ajustement du pH et 40-85 % en poids de l'au moins une charge, et
dans laquelle ledit produit comprend de la cellulose microcristalline en la quantité de 30-70 % en poids.

2. Produit selon la revendication 1, dans lequel la source de nicotine a été dérivée par extraction à partir de tabac ou par synthèse chimique.

3. Produit selon la revendication 1 ou 2, dans lequel la source de nicotine est stabilisée sous la forme d'un sel de nicotine ou liée à une résine échangeuse d'ions.

4. Produit selon la revendication 3, dans lequel la résine échangeuse d'ions est une résine échangeuse de cations faibles, telle que le polacrilex.

5. Produit selon les revendications 1-3, dans lequel la source de nicotine est le benzoate de nicotine ou le maléate de nicotine.

6. Produit selon l'une quelconque des revendications précédentes, dans lequel l'au moins un agent d'ajustement du pH est un tampon comprenant carbonates, bicarbonates, borates, glycinates, ammonium, phosphates, hydroxides, tris, ou leurs mélanges.

7. Produit selon l'une quelconque des revendications précédentes, dans lequel ladite au moins une charge comprend des polyalcools, des édulcorants, des polysaccharides, une cellulose, des fibres naturelles, des arômes, ou leurs mélanges.

8. Produit selon l'une quelconque des revendications précédentes, dans lequel l'au moins une charge comprend une cellulose microcristalline, telle qu'Avicel PH-200.

9. Produit selon la revendication 7, dans lequel les édulcorants sont choisis dans le groupe constitué par le mannitol, le xylitol, le maltitol, le sucralose, l'acésulfame potassique, l'aspartame, les glycosides de stéviol, ou leurs mélanges.

10. Produit selon la revendication 7, dans lequel les arômes sont choisis dans le groupe constitué par menthol, cannelle, menthe, menthe poivrée, menthe verte, pomme, cerise, melon, mangue, pêche, fruit de la passion, orange, orange sanguine, pamplemousse, mandarine, tangerine, réglisse, gingembre, vanille, gaulthérie, citron, citron vert, fraise, framboise, mûre de saumon, airelle rouge, canneberge, myrtille, baie muscadine, argousier, chocolat, café, moka ou leurs mélanges.

11. Produit selon l'une quelconque des revendications précédentes, dans lequel les proportions en poids de la composition en poudre sont ; 0,5-2 % en poids de la source de nicotine calculés en tant que nicotine base, 4-15 % en poids de l'agent d'ajustement du pH et 70-85 % en poids de l'au moins une charge.

12. Produit selon l'une quelconque des revendications précédentes, dans lequel la poche a une fraction de libération de nicotine de 50-100 % après 30 min.

13. Produit selon l'une quelconque des revendications précédentes, dans lequel la poche est faite d'un matériau non tissé.

14. Procédé pour la préparation du produit selon l'une quelconque des revendications 1-13, qui comprend les étapes de :
a) mise à disposition de la source de nicotine, de l'au moins un agent d'ajustement du pH et de l'au moins une charge, sous forme de poudre ;
b) mélange des poudres mises à disposition à l'étape a) ;
c) éventuellement tamisage du mélange de poudres de l'étape b) en une composition en poudre ; et
d) éventuellement remplissage de la composition en poudre dans une poche.

15. Utilisation non thérapeutique du produit selon l'une quelconque des revendications 1-13 pour l'administration orale de nicotine.
